# FASCICULE DE BREVET EUROPEEN

(11) **EP 4 440 593 B1**
(45) Date de publication et mention de la délivrance du brevet: **24.06.2026**
(21) Numéro de dépôt: 22822489.5
(22) Date de dépôt: 28.11.2022
(51) Int. Cl.: A61K 38/05, A61K 38/06, A61K 38/14, A61K 35/747, A61P 5/06

(54) **UTILISATION D'UN MURAMYL PEPTIDE DANS LE TRAITEMENT DES RETARDS DE CROISSANCE SQUELETTIQUE**
VERWENDUNG EINES MURAMYLPEPTIDS ZUR BEHANDLUNG VON SKELETTWACHSTUMSVERZÖGERUNG
USE OF A MURAMYL PEPTIDE IN THE TREATMENT OF SKELETAL GROWTH RETARDATION

(30) Priorité: 29.11.2021 FR 2112679
(43) Date de publication de la demande: 09.10.2024
(73) Titulaire: Université Claude Bernard Lyon I, 69100 Villeurbanne (FR); Centre National de la Recherche Scientifique, 75016 Paris (FR); Ecole Normale Supérieure de Lyon, 69007 Lyon (FR); Institute of Microbiology of the Czech Academy of Sciences, 14220 Praha 4 (CZ)
(72) Inventeur: LEULIER, François, 69360 Solaize (FR); SCHWARZER, Martin, 54922 Nový Hrádek (CZ)
(74) Mandataire: IPAZ
(86) Numéro de dépôt international: PCT/EP2022/083416
(87) Numéro de publication internationale: WO 2023/094644

(56) Documents cités:
- WO-A1-2017/223337
- WO-A1-2019/023555
- WO-A1-93/13791
- FR-A1- 3 020 949
- JP-A- 2003 125 758
- US-A1- 2015 141 352

## Description

### Domaine technique

La présente invention concerne l'utilisation d'au moins un muramyl peptide, tel que le muramyl dipeptide ou l'un de ses analogues, dans le traitement des retards de croissance squelettique chez un sujet juvénile, notamment chez des humains ou des animaux en sous-nutrition.

### Etat de la technique

La croissance linéaire et pondérale est une capacité inhérente à tous les organismes multicellulaires juvéniles. Chez les mammifères, la croissance postnatale est contrôlée par l'axe somatotrope où l'hormone de croissance (GH, de l'anglais « *Growth Hormone* ») ordonne au foie et aux tissus périphériques de produire le facteur de croissance analogue à l'insuline de type 1 (IGF-1, de l'anglais « *Insuline-like Growth Factor-1* »), afin de promouvoir la croissance systémique, c'est-à-dire la croissance coordonnée de ses organes, ses tissus et son squelette. Ainsi l'IGF-1 est le facteur principal contrôlant la croissance squelettique appelée aussi croissance linéaire ou croissance longitudinale. Après l'IGF-1, l'insuline est la principale hormone anabolisante de l'organisme qui régule le métabolisme des glucides, des lipides et des protéines. Si l'axe somatotrope (GH/IGF-1) est d'une importance capitale pendant la croissance, une régulation coordonnée du métabolisme par l'insuline est nécessaire pour fournir des substrats et des carburants aux tissus en croissance. Par conséquent, l'insuline influence également le développement et la croissance des animaux préférentiellement via un soutien à la croissance pondérale.

Un retard de croissance juvénile est caractérisé par un taux de croissance réduit pendant le développement postnatal. Il se manifeste par une réduction du poids (croissance pondérale) et une diminution de la taille de l'individu (croissance squelettique, appelée également croissance linéaire ou croissance longitudinale) à un âge donné par rapport à la population saine au même âge. Il s'agit d'une manifestation primaire liées à une dénutrition aigüe ou à une sous-nutrition chronique et/ou à des infections entériques récurrentes, telles que la diarrhée et les helminthiases, dans la petite enfance et même avant la naissance, en raison de la malnutrition de la mère pendant le développement du fœtus. En 2012, on estimait que 162 millions d'enfants de moins de 5 ans, soit 25 %, souffraient d'un retard de croissance. Plus de 90 % des enfants souffrant d'un retard de croissance dans le monde vivent en Afrique et en Asie, où respectivement 36 % et 56 % des enfants sont touchés (United Nations Children's Fund, World Health Organization, The World Bank. UNICEFWHO- World Bank Joint Child Malnutrition Estimates). Une fois établi, le retard de croissance et ses effets systémiques persistent généralement jusque l'âge adulte et perdurent.

Les muramyl peptides sont des constituants des peptidoglycanes des parois cellulaires bactériennes qui sont libérés dans l'organisme lors de la dégradation, la croissance ou la division des bactéries constituant le microbiote intestinal. Ils sont considérés comme des régulateurs naturels de l'immunité. Les muramyl peptides, selon de nombreuses études, sont les fragments minimaux biologiquement actifs qui initient la réponse immunitaire après avoir interagi avec les récepteurs intracellulaires de la famille NLR (de l'expression anglaise « *NOD-like Receptors* ») de l'immunité innée (NOD1, NOD2, NALP3, etc.). Parmi ceux-ci, le muramyl dipeptide (MDP, N-acétylmuramyl-L-alanyl-isoglutamine) est une molécule synthétique immunoréactive constitué d'acide N-acétyl-muramique attaché à une courte chaîne d'acides aminés de -Ala-D-isoGln. Il a été identifié pour la première fois dans le peptidoglycane de la paroi cellulaire bactérienne comme un composant actif de l'adjuvant complet de Freund (FCA). En 1974, on a découvert que le MDP était la structure minimale requise pour l'efficacité du FCA, l'un des adjuvants les plus puissants et les plus largement utilisés dans les modèles expérimentaux animaux. Il a été prouvé que le MDP et ses dérivés présentent des propriétés immunomodulatrices significatives, via l'un des PRR (« *Pattern Recognition Receptors* »), le récepteur NOD2 (de l'expression anglaise « *Nucleotide-binding Oligomerization Domain 2* ») (Girardin S. et al.,. J. Biol. Chem., 2003, 278(11), p. 8869- 72 ; F. Coulombe et al, 2012 PloS ONE, 7(5) : Article ID e36734). Il est également connu que les muramyl dipeptides activent les macrophages et d'autres cellules du système immunitaire pour tuer les cellules cancéreuses (Z. Jakopin, Current Medicinal Chemistry, 2013, 20(16), 2068- 2079).

Par ailleurs, la demande de brevet US 2015/141352 décrit l'utilisation du muramyl-dipeptide (MDP) ou d'une composition pharmaceutique le contenant pour prévenir la perte osseuse, promouvoir la régénération osseuse et la formation de l'os. Parmi les désordres osseux pouvant être traités par administration systémique de MDP figure notamment le rachitisme qui est une maladie de la croissance et de l'ossification observée chez le nourrisson et le jeune enfant en état de sous nutrition aigue principalement due à une carence en vitamine D et en calcium. Le rachitisme se caractérise par un retard de la croissance pondérale et une insuffisance de calcification des os et des cartilages.

La demande de brevet FR3020949 décrit une composition comprenant une souche de Lactobacillus à tropisme intestinal pour une utilisation visant à favoriser la croissance juvénile en cas de malnutrition.

Bien que de nombreuses applications thérapeutiques du muramyl dipeptide ou de ses dérivés aient déjà été proposées, son utilisation pour le traitement des retards de la croissance squelettique chez des humains ou des animaux juvéniles en sous-nutrition chronique n'a pour l'heure jamais été décrite.

La présente invention s'inscrit justement dans le rétablissement de conditions permettant de restaurer une meilleure croissance squelettique, voire de restaurer une croissance squelettique normale (appelée aussi linéaire ou longitudinale), notamment dans un contexte de sous-nutrition chronique, et en particulier dans une situation de carence protéique.

C'est dans ce contexte qu'il a été découvert de façon surprenante que des muramyl peptides, et en particulier le muramyl dipeptide (MDP) et ses analogues chimiques, pouvaient avantageusement être utilisés, notamment par voie orale ou respiratoire, dans le traitement des retards de croissance squelettique chez des humains ou des animaux juvéniles, notamment lorsque ces retards de croissance sont dus à une sous-nutrition chronique, en particulier à une carence protéique, c'est-à-dire à un apport journalier en protéines inférieur aux apports nutritionnels conseillés (ANC). A titre indicatif, les ANC en protéines chez les enfants entre 0 et 36 mois sont de l'ordre de 9 à 12 g/jour, de 15 à 30 g/jour pour les enfants de 4 à 10 ans et de 30 à 50 g/jour pour les garçons de 11 à 18 ans et de 30 à 40 g/jour pour les filles de 11 à 18 ans. Il a ainsi pu être démontré que l'administration orale de MDP, ou de l'un de ses analogues tels que le mifamurtide ou le murabutide, ont un effet positif sur la croissance squelettique (linéaire ou longitudinale) dans un modèle de souris juvéniles soumises à un régime nutritionnel carencé en protéines et mimant une sous-nutrition chronique (exemples 1 et 2).

La présente invention a ainsi pour objectif de proposer de nouvelles compositions permettant d'améliorer une croissance squelettique (linéaire ou longitudinale) chez des sujets juvéniles, humains ou animaux, voire de rétablir une croissance normale, notamment chez les humains ou les animaux ayant été ou étant soumis à une sous-nutrition chronique, et en particulier à une carence en protéines.

Un autre objectif de l'invention est de fournir de telles compositions, pouvant aussi s'inscrire dans un contexte thérapeutique.

### Objets de l'invention

La présente invention est définie dans les revendications. La présente invention a pour premier objet une composition comprenant au moins un muramyl peptide, pour une utilisation dans le traitement des retards de croissance squelettique (ou croissance linéaire ou croissance longitudinale) chez un humain ou un animal juvénile.

Selon ce premier objet, la composition peut en outre renfermer au moins un adjuvant permettant de favoriser la croissance, notamment juvénile, ledit adjuvant pouvant en particulier être choisi parmi des souches bactériennes ayant un tropisme intestinal, notamment des souches d'espèces commensales ou d'espèces présentes dans le microbiote intestinal de l'espèce cible, ou acceptables en tant que probiotique.

Ainsi, l'indication de la composition conforme à la présente invention peut être thérapeutique ou santé, comme médicament ou comme composition pharmaceutique, ou nutritionnelle, notamment comme complément alimentaire, ou postbiotique, voire probiotique lorsque ladite composition renferme au moins une souche bactérienne à tropisme intestinal.

Un deuxième objet de l'invention est l'utilisation non thérapeutique d'une composition conforme à l'invention comprenant au moins un muramyl peptide et au moins une bactérie appartenant aux familles *Lactobacillaceae, Streptoccaceae, Enterococcaceae, Leuconostocaceae,* et *Bifidobacteriaceae* à titre d'adjuvant, ladite bactérie étant vivante ou inactivée, dans une méthode de traitement probiobique non thérapeutique pour favoriser la croissance squelettique chez un sujet animal ou humain.

### Définitions

Un analogue ou analogue chimique du muramyl dipeptide est une espèce chimique qui ne diffère du muramyl dipeptide que par le remplacement d'un atome ou d'un groupe d'atomes par un autre, et qui présente des propriétés physicochimiques et biologiques voisines de celles du muramyl dipeptide.

La définition d'un retard de croissance squelettique juvénile selon l'Organisation mondiale de la santé (OMS) est que la valeur de la "taille pour l'âge" est inférieure à deux écarts types de la médiane des normes de croissance de l'enfant sain.

Un sujet juvénile est un sujet n'ayant pas atteint la maturité sexuelle.

Dans la présente description, les expressions « croissance squelettique », « croissance longitudinale » et « croissance linéaire » sont synonymes de « croissance juvénile ». Cette croissance peut être basiquement mesurée par la taille chez un humain ou par la taille du museau ou de la bouche à la base de la queue chez les animaux mammifères ou par la longueur du corps chez les vertébrés et les invertébrés.

La « croissance juvénile » ne comprend pas la croissance pondérale, que celle-ci soit rapportée à une augmentation de la masse musculaire et/ou à une augmentation de la masse graisseuse. A l'inverse, la croissance juvénile au sens de la présente invention peut être évaluée par une augmentation de la longueur des os ou de la longueur du corps et/ou une augmentation de la production d'IGF-1 et/ou de ses analogues fonctionnels pour les animaux pourvus d'un exosquelette mais aussi dépourvus de squelette osseux comme les invertébrés, et tels que des peptides appartenant à la famille des facteurs apparentés à l'insuline (« *insulin-like peptides* » et « *insulin-like growth factors* »).

La sous-nutrition (ou sous-alimentation) est un état de manque important de nourriture caractérisé par un apport alimentaire insuffisant pour combler les besoins nutritionnels et les dépenses énergétiques journalières d'un individu et entraînant des carences nutritionnelles et métaboliques. La sous-nutrition peut être chronique ou aigue. Dans le cas de sous nutrition chronique, et en particulier dans le cas de carence en protéines, les sujets sont en état dit de « *stunting* » ou de « *short stature* », c'est-à-dire en état de retard de croissance altéré voire irréversible, contrairement aux cas de sous nutrition aigue (comme le rachitisme) où le retard de croissance, notamment pondérale, peut être réversible.

### Description détaillée

Le premier objet de l'invention est une composition comprenant au moins un muramyl peptide, pour une utilisation dans le traitement des retards de croissance squelettique chez un humain ou un animal juvénile.

Selon une forme de réalisation particulière et préférée de l'invention, ledit humain ou animal est en sous-nutrition chronique. De façon tout à fait préférentielle, cet état de sous-nutrition chronique est une carence protéique.

Selon une forme de réalisation préférée de l'invention, ledit au moins un muramyl peptide est choisi parmi le muramyl dipeptide (ou MDP ou MurNAc-L-Ala-γ-D-Glu, N° CAS 53678-77-6) et les analogues du muramyl dipeptide.

Le MDP est disponible dans le commerce sous forme lyophilisée (poudre), notamment auprès de la société InvivoGen Europe (Toulouse, France). Il est d'origine bactérienne et peut se présenter sous forme purifiée ou semi-purifiée telle que par exemple sous forme de MDP lié à des fragments de paroi cellulaire bactérienne.

Les analogues du muramyl dipeptide sont des peptides synthétiques parmi lesquels on peut notamment citer le mifamurtide (ou muramyl tripeptide phosphatidyl éthanolamine, également dénommé MTP-PE) tel que par exemple le produit vendu sous la dénomination commerciale Mepact, par la société Takeda, le murabutide (ou muramyl dipeptide butyl ester) (InvivoGen Europe, Toulouse, France), la 6-O-stearoyl-N-acetyl-muramyl-L-alanyl-D-isoglutamine (ou L18-MDP), le muropeptide MurNAc-Ala-D-isoGln-Ly (M-TriLYS, InvivoGen Europe, Toulouse, France) et leurs mélanges.

Selon une forme de réalisation préférée de l'invention, l'analogue du muramyl dipeptide est choisi parmi le murabutide et le mifamurtide.

Selon une forme de réalisation préférée de l'invention, ledit au moins un muramyl peptide est le muramyl dipeptide.

Ledit au moins un muramyl peptide représente généralement de 10 µg à 100 mg environ par dose unitaire de composition, plus particulièrement de 50 µg à 50 mg environ, préférentiellement de 100 µg à 10 mg µg environ, et encore plus préférentiellement de 0,5 à 1 mg par dose unitaire de composition.

Le nombre de doses à administrer et la fréquence d'administration de ces doses, sont bien entendu à ajuster en fonction de la taille, du poids du sujet humain ou animal à traiter, ainsi que de la sévérité du retard de croissance et de la biodisponibilité du ou des muramyl peptides administrés.

Selon la présente invention, on entend par dose unitaire, la quantité de composition conforme à l'invention susceptible d'être administrée, par prise, à un sujet à traiter, quelle que soit la masse totale de cette dose unitaire.

Selon une forme de réalisation particulière et préférée de l'invention, la composition comprend en outre au moins un adjuvant permettant de favoriser la croissance, notamment chez un sujet juvénile.

Selon cette forme de réalisation particulière, ledit adjuvant peut être choisi parmi des souches bactériennes ayant un tropisme intestinal, des constituants bactériens, des postbiotiques et leurs mélanges.

Par bactérie ayant un « tropisme intestinal », on entend une bactérie ayant la capacité de passer la barrière gastrique et qui est capable de persister dans les intestins.

Conformément à une caractéristique avantageuse de l'invention, ladite bactérie peut favoriser la production d'IGF-1 chez l'homme ou l'animal qui est traité par la composition conforme à l'invention.

Les souches bactériennes ayant un tropisme intestinal utilisables dans la composition conforme à l'invention sont notamment des souches d'espèces commensales ou d'espèces présentes dans le microbiote intestinal de l'espèce cible ou acceptables en tant que probiotique.

Définis en 2001 par l'Organisation mondiale de la Santé (OMS) et l'Organisation des Nations Unies pour l'alimentation et l'agriculture (FAO), les probiotiques sont des micro-organismes vivants qui, lorsqu'ils sont ingérés en quantité suffisante exercent des effets positifs sur la santé, au-delà des effets nutritionnels habituels.

Selon l'invention, les postbiotiques sont des composés inactivés à base de cellules microbiennes/bactériennes inactivées ou de composants cellulaires isolés inactivés (de type acides gras à chaîne courte, peptides, protéines, composant des parois bactériennes ou encore enzymes), avec ou sans métabolites, qui offrent également des bienfaits à la santé.

Les souches bactériennes sont choisies parmi les bactéries appartenant aux familles suivantes : *Lactobacillaceae, Streptoccaceae, Enterococcaceae, Leuconostocaceae,* et *Bifidobacteriaceae.*

Parmi de telles souches, on peut plus particulièrement citer les souches du genre *Lactobacillus,* en particulier de l'une des espèces suivantes, *Lactobacillus delbrueckii, Lactobacillus plantarum* (ou selon la nouvelle appellation *Lactiplantibacillus plantarum), Lactobacillus fermentum, Lactobacillus casei, Lactobacillus paracasei,* et *Lactobacillus rhamnosus*.

Plus particulièrement, il s'agit de bactéries appartenant aux espèces *Lactobacillus plantarum, Lactobacillus fermentum, Lactobacillus casei, Lactobacillus paracasei,* et *Lactobacillus rhamnosus*. Suivant une modalité, la souche bactérienne est choisie parmi les espèces *Lactobacillus plantarum, Lactobacillus fermentum,* et *Lactobacillus casei.*

Suivant un mode de réalisation, la souche bactérienne est choisie parmi *L. plantarum* WJL déposée à la CNCM (Collection Nationale de Cultures de Micro-organismes - Institut Pasteur) le 15 février 2017 sous le numéro I-5169, *L. plantarum* IGFL1 déposée le 19 Juillet 2017 à la CNCM sous le numéro I-5217, *L. plantarum* IGFL2 déposée le 19 Juillet 2017 à la CNCM sous le numéro I-5218, *L. plantarum* G821 déposée le 11 mai 2015 à la CNCM sous le numéro I-4979, *L. plantarum* NIZO2877, *L. casei* ATCC 393, *L. casei* L919, *L. paracasei* ATCC25302, *L. paracasei Shirota, L. fermentum* ATCC9338, *L. rhamnosus* L900, *L. rhamnosus* L908, *L. rhamnosus* GG, et leurs mélanges.

Parmi de telles souches, on préfère *L. plantarum WJL* déposée à la CNCM le 15 février 2017 sous le numéro I-5169, *L. plantarum* IGFL1 déposée à la CNCM sous le numéro d'enregistrement I-5217 le 19 Juillet 2017, *L. plantarum* G821 déposée à la CNCM sous le numéro d'enregistrement I-4979 le 11 mai 2015, *L. plantarum* IGFL2 déposée à la CNCM sous le numéro d'enregistrement I-5218 le 19 Juillet 2017, et *L. plantarum* NIZO2877.

La souche IGFL1 a été obtenue par évolution expérimentale (c.à.d. par sélection de variants naturels ayant accumulés des mutations induites naturellement lors des processus de réplication de l'ADN) de la souche WJL.

La souche G821, a été obtenue par évolution expérimentale (c.à.d. par sélection de variants naturels ayant accumulés des mutations induites naturellement lors des processus de réplication de l'ADN) de la souche NIZ02877.

La souche IGFL2 a été obtenue par évolution expérimentale (c.à.d. par sélection de variants naturels ayant accumulés des mutations induites naturellement lors des processus de réplication de l'ADN) de la souche NIZ02877.

D'autres exemples de souches appropriées sont les suivantes : *L. casei* ATCC 393, *L. casei* L919 (Koryszewska-Baginska A. *et al.,* 26 septembre 2013, Genome Announc), *L. paracasei* ATCC25302, *L. paracasei* Shirota (Yuki N et al., Int J Food Microbiol. 1er avril 1999; 48(1 ) :51 -7), *L. fermentum* ATCC9338, *L. rhamnosus* L900 (Aleksandrzak-Piekarczyk T. et al., Genome Announc, 15 août 2013), *L. rhamnosus* L908 (Koryszewska-Baginska A. *et al.,* 20 février 2014, Genome Announc), *L. rhamnosus* GG (Kankainen M. et al., Proc Natl Acad Sci USA, 6 octobre 2009).

La composition conforme à l'invention peut comprendre la ou les souches bactériennes sous forme vivante. Il peut s'agir d'une suspension bactérienne, qui peut être congelée et décongelée avant utilisation, ou encore une poudre lyophilisée, pouvant être utilisée telle quelle ou après réhydratation dans un véhicule liquide approprié. Elle peut alors comprendre un excipient de lyophilisation classique.

Au sein de la composition conforme à la présente invention, la ou les souches bactériennes peuvent être présentes en une quantité d'environ 10⁵ à 10¹², notamment d'environ 10⁶ à 10¹², de préférence d'environ 10⁸ à 10¹² cellules bactériennes formant des colonies (CFU, de l'expression anglaise « *Colony forming Unit* »), par gramme de composition.

La composition conforme à l'invention peut également comprendre la ou les souches bactériennes sous forme inactivée. L'inactivation des souches bactérienne peut être réalisée par toute technique adaptée et bien connue de l'homme du métier, telle que par exemple par un traitement chimique bactériolytique ou bactériostatique ou des traitements physiques par pression, rayonnements, chaleur ou ultrasons.

Lorsque la composition conforme à l'invention renferme, à titre d'adjuvant, des constituants bactériens, ceux-ci peuvent par exemple être choisis parmi des bactéries inactivées, des lysats bactériens, des parois bactériennes purifiées et des fragments de parois bactériennes purifiées de bactéries ayant un tropisme intestinal, des peptidoglycanes et des acides téichoïques. Les bactéries à tropisme intestinal utilisables pour préparer des parois bactériennes purifiées ou des fragments de parois bactériennes purifiées peuvent notamment être choisies parmi les souches bactériennes ayant un tropisme intestinal telles que décrites ci-dessus.

Selon une forme de réalisation particulière et préférée de l'invention, la composition comprend des parois bactériennes purifiées de *L. plantarum* WJL et/ou des fragments de parois bactériennes purifiées de *L. plantarum* WJL.

Au sein de la composition conforme à la présente invention, le ou les constituants bactériens peuvent être présents en une quantité d'environ 0,02 µg à 200 mg, notamment d'environ 0,2 µg à 2 mg, de préférence d'environ 20 µg, par dose unitaire de composition.

La composition conforme à l'invention peut en outre renfermer, à titre d'ingrédient additionnel, un ou plusieurs compléments alimentaires et/ou nutriments ceux-ci peuvent notamment être choisis parmi les glucides, les lipides, des peptides autres que les muramyl peptides, les protéines, les acides gras, les acides aminés tels que la L-glutamine, les vitamines, les minéraux, et les prébiotiques tels que par exemple les fibres de gomme d'acacia, etc...

La composition conforme à la présente invention peut être une forme d'administration par voie orale ou respiratoire et se présenter par exemple sous la forme d'une poudre, d'une gélule ou d'un comprimé, d'une solution buvable, d'un spray nasal ou encore d'un spray aérien. Selon une forme de réalisation particulière de l'invention, ladite forme d'administration est sous forme gastro-résistante. Cette forme de réalisation est particulièrement adaptée lorsque la composition renferme au moins une souche bactérienne à titre d'adjuvant en plus dudit au moins muramyl peptide. En effet, cette forme de présentation lui permet de passer l'estomac sans être dégradée puis de libérer ladite bactérie au niveau de l'intestin.

La présente invention a également pour objet l'utilisation d'une composition comprenant au moins un muramyl peptide et au moins une bactérie appartenant aux familles *Lactobacillaceae, Streptoccaceae, Enterococcaceae, Leuconostocaceae,* et *Bifidobacteriaceae* à titre d'adjuvant, ladite bactérie étant vivante ou inactivée, dans une méthode de traitement probiobique non thérapeutique pour favoriser la croissance squelettique chez un sujet animal ou humain.

Selon l'utilisation conforme à l'invention, ladite méthode comprend l'administration d'une quantité suffisante d'une composition conforme au premier objet de l'invention et telle que définie ci-dessus. La quantité, la fréquence d'administration dépendront notamment de la sévérité du retard de croissance squelettique, de l'âge et de l'état du patient ou de l'animal. La méthode comprendra l'administration en une ou plusieurs fois, pouvant être échelonnée sur la période de croissance du sujet (jusqu'à la puberté ou la maturité sexuelle), de doses de composition selon l'invention. Les doses peuvent en particulier être fractionnées pour faciliter l'administration, notamment en fonction de l'âge du sujet. La fréquence d'administration est notamment comprise entre une dose (unique ou fractionnée) tous les jours et une dose tous les mois. Typiquement, la fréquence d'administration sera comprise entre une dose (unique ou fractionnée) tous les jours et une dose toutes les semaines, voire tous les 2, 3, 4, 5 ou 6 jours. Chaque dose (unique ou fractionnée) représente notamment plusieurs grammes à plusieurs dizaines de grammes de composition.

D'autres particularités et avantages de l'invention ressortiront de la description détaillée des exemples qui suivent, ainsi que des figures 1 à 14 annexées qui représentent les différents effets d'un traitement sur la croissance juvénile de jeunes souriceaux traités quotidiennement pendant 5 semaines après le sevrage par un placebo, du MDP ou du murabutide (Exemple 1 et figures 1 à 6) ou par un placebo ou du mifamurtide (Exemple 2 et figures 7 à 12), ou par un placebo, du MDP seul, une association de MDP et de bactéries *Lactiplantibacillus plantarum* souche WJL ou des *Lactiplantibacillus plantarum* souche WJL seules (exemple 3 et figures 13 et 14) et sur lesquelles :

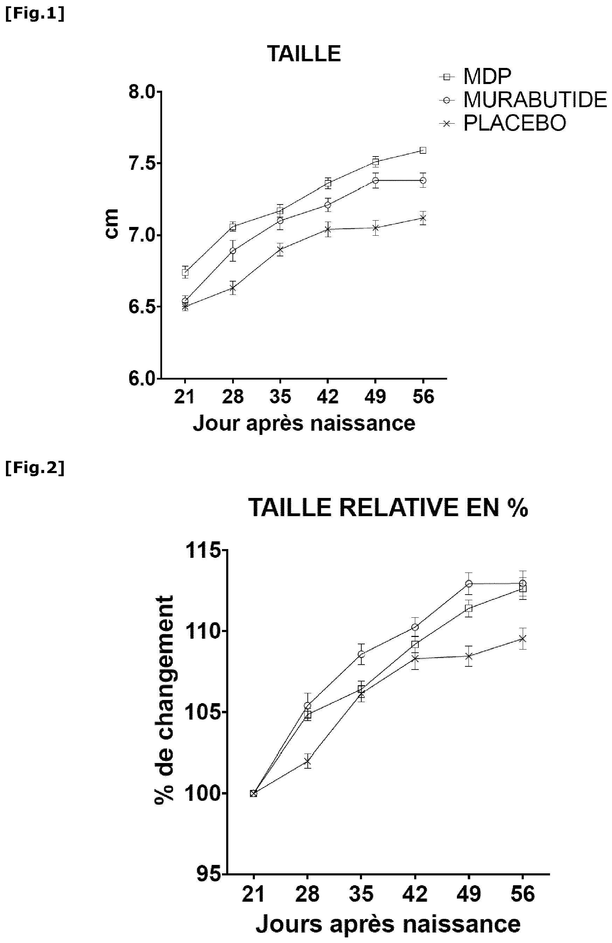

**[****Fig.1****]** : représente la taille (en cm) des animaux traités soit par le placebo, soit par le MDP, soit par le murabutide en fonction du nombre de jours après la naissance.
**[****Fig.2****]** : représente la taille relative (en %) des animaux traités soit par le placebo, soit par le MDP, soit par le murabutide en fonction du nombre de jours après la naissance.

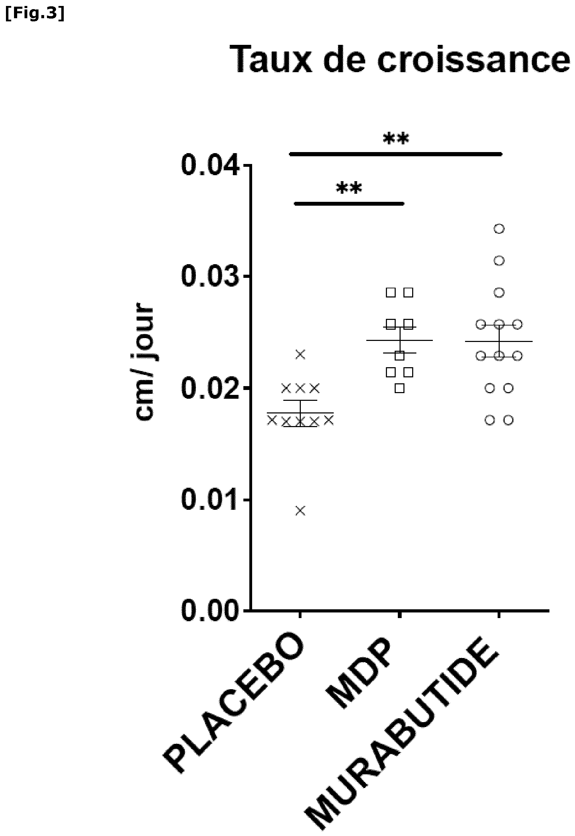

**[****Fig.3****]** : représente le taux de croissance (en cm/jour) des animaux traités soit par le placebo, soit par le MDP, soit par le murabutide.

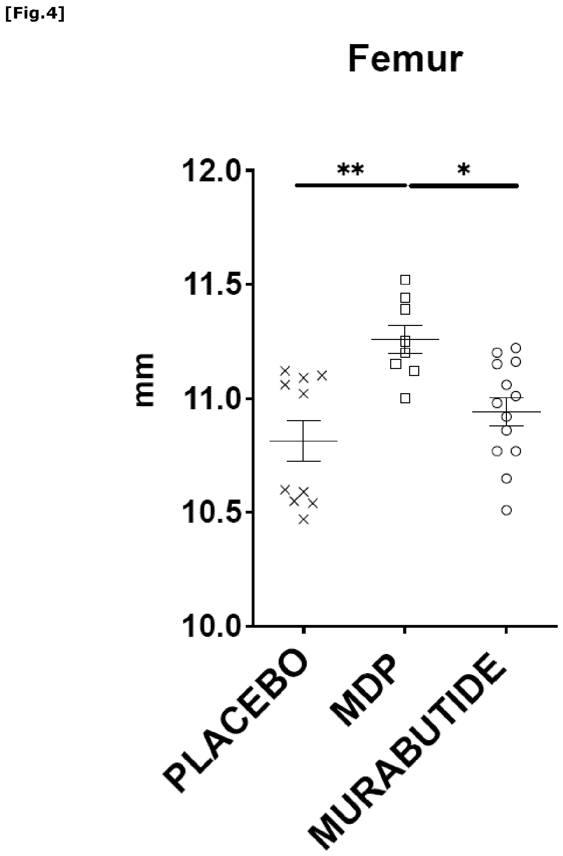

**[****Fig.4****]** : représente la taille du fémur (en mm) des animaux traités soit par le placebo, soit par le MDP, soit par le murabutide.

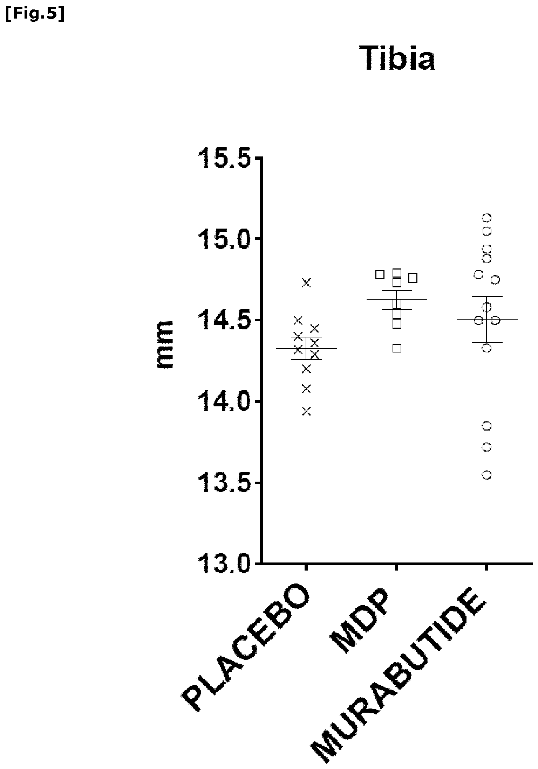

**[****Fig.5****]** : représente la taille du tibia (en mm) des animaux traités soit par le placebo, soit par le MDP, soit par le murabutide.

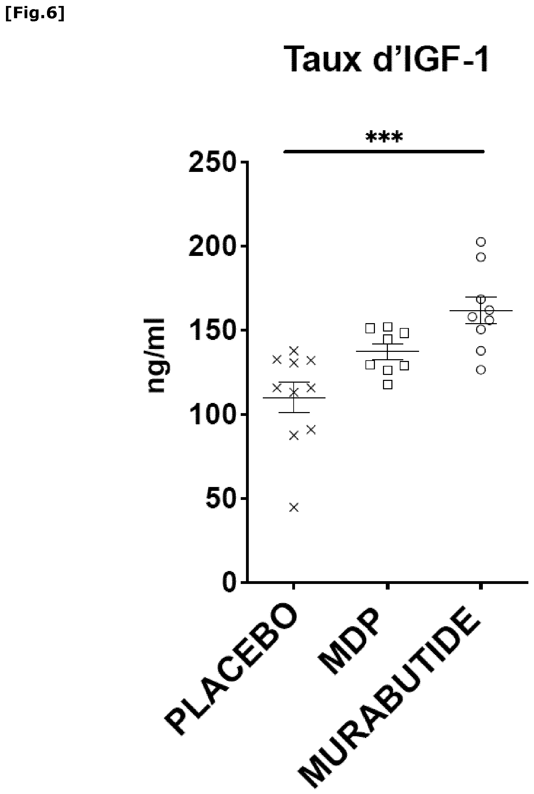

**[****Fig.6****]** : représente la quantité circulante du facteur de croissance IGF-1 (en ng/ml) des animaux traités soit par le placebo, soit par le MDP, soit par le murabutide.

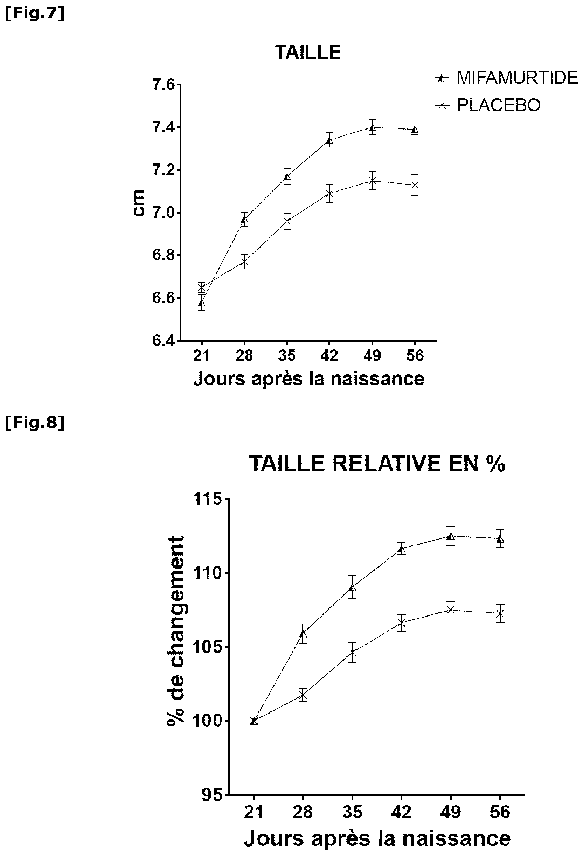

**[****Fig.7****]** : représente la taille (en cm) des animaux traités soit par le placebo, soit par le mifamurtide en fonction du nombre de jours après la naissance.
**[****Fig.8****]** : représente la taille relative (en %) des animaux traités soit par le placebo, soit par le mifamurtide en fonction du nombre de jours après la naissance.

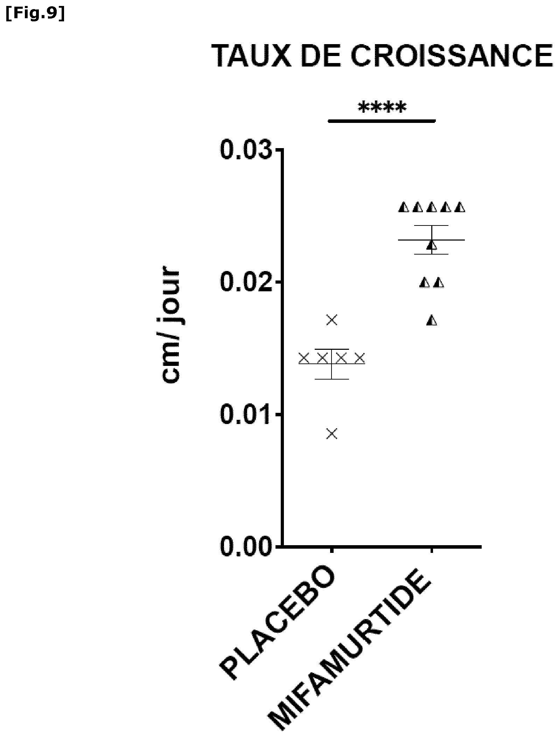

**[****Fig.9****]** : représente le taux de croissance (en cm/jour) des animaux traités soit par le placebo, soit par mifamurtide.

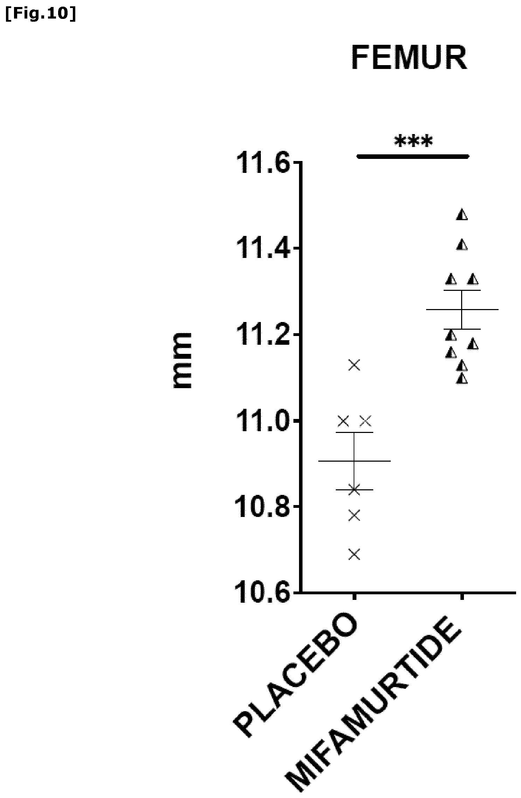

**[****Fig.10****]** : représente la taille du fémur (en mm) des animaux traités soit par le placebo, soit par le mifamurtide.

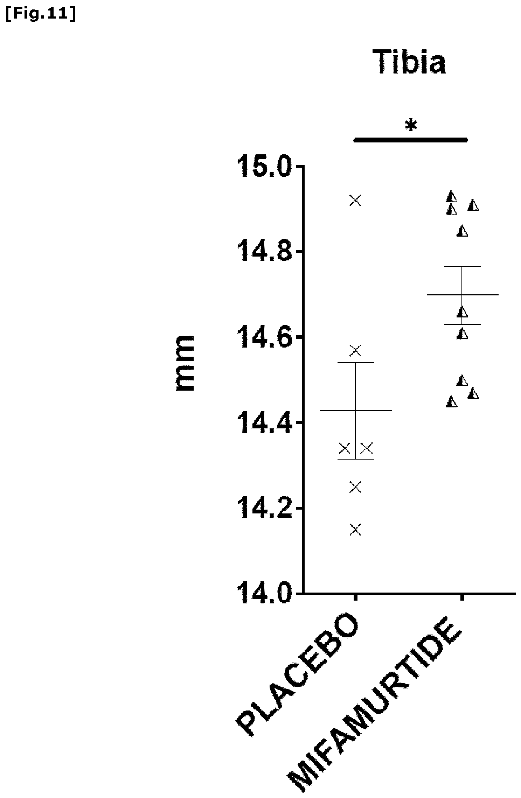

**[****Fig.11****]** : représente la taille du tibia (en mm) des animaux traités soit par le placebo, soit par le mifamurtide.

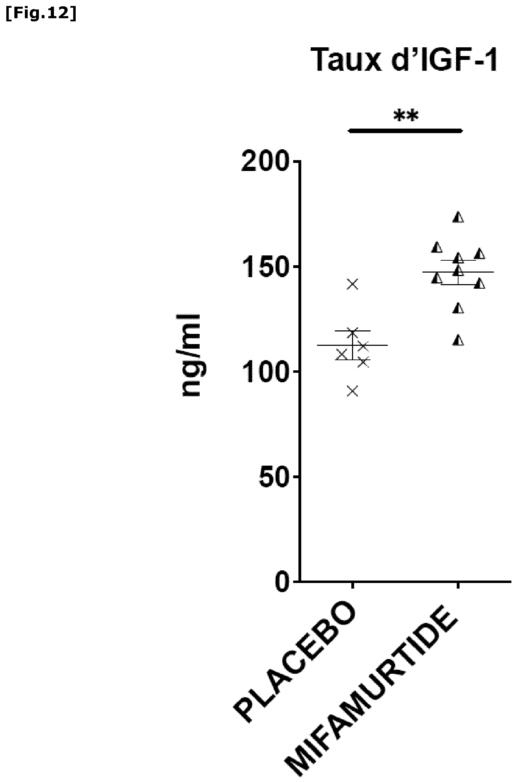

**[****Fig.12****]** : représente la quantité circulante du facteur de croissance IGF-1 (en ng/ml) des animaux traités soit par le placebo, soit par le mifamurtide.

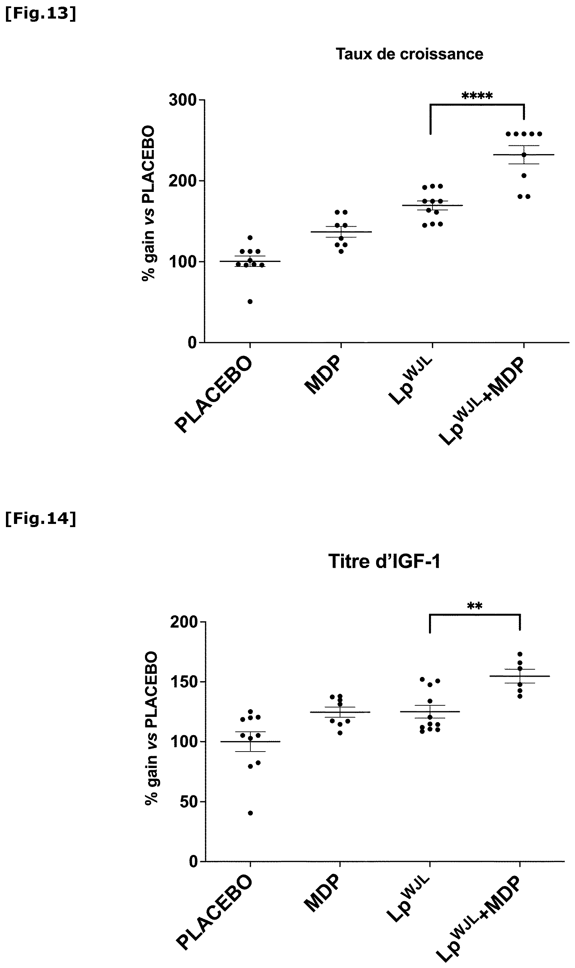

**[****Fig.13****]** : représente le gain de croissance (en %) chez les animaux traités soit par le MDP seul, soit par les bactéries *Lactiplantibacillus plantarum* souche WJL seules, soit par l'association MDP + bactéries *Lactiplantibacillus plantarum* souche WJL, comparativement aux animaux traités par le placebo.
**[****Fig.14****]** : représente le pourcentage d'amélioration du taux d'IGF-1 circulant (en %) chez les animaux traités soit par le MDP seul, soit par les bactéries *Lactiplantibacillus plantarum* souche WJL seules, soit par l'association MDP + bactéries *Lactiplantibacillus plantarum* souche WJL, comparativement aux animaux traités par le placebo.

### EXEMPLES

Les matières premières utilisées dans les exemples sont les suivantes :
- Muramyl dipeptide vendu sous la dénomination MDP par la société InvivoGen Europe, 5, rue Jean Rodier, F-31400, Toulouse, France ;
- Murabutide, vendu sous la dénomination Murabutide par la société InvivoGen Europe, 5, rue Jean Rodier, F-31400 Toulouse France. ;
- Mifamurtide, vendu sous la dénomination Mifamurtide par la société CliniSciences, 74 Rue des Suisses, 92000 Nanterre, France. ;
- Diméthylsulfoxyde (DMSO), vendu sous la dénomination Dimethyl sulfoxide par la société Merck, Darmstadt, Germany.
- Maltodextrine, vendue sous la dénomination Maltodextrin par la société Merck, Darmstadt, Germany
- Bactéries *Lactiplantibacillus plantarum* souche WJL (déposée à la CNCM le 15 février 2017 sous le numéro 1-5169).

### EXEMPLE 1 : Mise en évidence des effets de l'administration de différents muramyl peptides dans un modèle de souris en sous-nutrition chronique

Dans cet exemple, on a testé les effets plusieurs compositions conformes à la présente invention comprenant des quantités isomolaires de MDP ou de murabutide (MDP M*asse* molaire moyenne en poids (Mw) =492,5 g/mol, Murabutide Mw = 548,6 g/mol) dans un modèle de souris en sous-nutrition chronique, à savoir :
- une composition A comprenant 25 µg de MDP dans 30 µL de composition placebo,
- une composition B comprenant 28 µg de murabutide dans 30 µL de composition placebo.

Ces compositions ont été comparées à une composition placebo ne renfermant qu'un cryoprotectant (Maltodextrine) resuspendu dans 30 µL de tampon salin stérile.

### 1.1 Préparation des compositions

15 µg de cryoprotectant ont été resuspendus dans 30 µL de tampon salin stérile pour générer une dose de composition placebo.

25 µg de MDP ont été resuspendus dans 30 µL de composition placebo pour obtenir la composition A à une concentration finale en MDP de 25 µg/ 30 µL.

28 µg de Murabutide ont été resuspendus dans 30 µL de composition placebo pour la composition B à une concentration finale en murabutide de 28 µg/ 30 µL (isomolaire de 25 µg/ 30 µL de MDP).

### 1.2 Préparation des animaux

Des souris de laboratoire C57Bl/6j conventionnelles ont été élevées au Laboratoire de Gnotobiologie pendant plus de 10 générations. Les souris ont été maintenues dans des cages IVC (Tecniplast, Italie), exposées à des cycles lumière-obscurité de 12:12 heures, alimentées en eau du robinet et nourries *ad libitum* avec le régime alimentaire stérile V1124-300 (Ssniff Spezialdiäten GmbH) (irradié ~25 kGy, Bioster, République Tchèque). Les souris ont été accouplées et après l'accouchement, la taille de la portée a été réduite à 6 petits par mère. Au 21^{ème} jour, les souris mâles ont été sevrées avec le régime expérimental pauvre en protéines et en graisses (3,5 kcal/gramme, protéines : 4,1 % en poids, glucides : 78% en poids, Graisse : 2% en poids) et suivies régulièrement jusqu'à 56 jours après la naissance (âge adulte chez la souris). Les souris sevrées avec le régime pauvre en protéines et en graisses ont été traitées 5 fois par semaine par le placébo, la composition A comprenant du MDP ou la composition B comprenant du Murabutide. A la fin de l'expérience, la nourriture et la litière ont été retirées à 8 heures du matin et les souris ont été sacrifiées après 5 heures de jeûne par inhalation d'isoflurane et dislocation cervicale. Tous les animaux ont été sacrifiés dans l'heure entre 13 et 14 heures.

### 1.3 Administration des compositions

La dose quotidienne de traitement était de 30 µL de placebo ou pour le MDP de 25 µg dans 30 µL de placebo et pour le murabutide de 28 µg dans 30 µL de placebo. Chacune des compositions a été administrée à la pipette sur la langue de la souris. La souris a été maintenue jusqu'à ce que la composition ait été avalée en totalité.

### 1.4 Description des tests effectués

Pour la mesure de la longueur du corps, les souris ont été brièvement anesthésiées à l'isoflurane (Piramal Healthcare, UK). La souris anesthésiée a été tenue par la queue et la longueur nez-anus a été mesurée à l'aide d'une règle. La longueur relative a été calculée comme un gain de longueur en %, où la longueur corporelle au jour 21 (sevrage) a été fixée à 100 %. Le taux de croissance de la longueur a été calculé en divisant le gain de longueur absolu du jour 21 au jour 56 par le nombre de jours (c'est-à-dire 35). Les os du fémur et du tibia ont été disséqués au moment du sacrifice, fixés dans du paraformaldéhyde à 4% - PBS pendant une nuit à 4°C, lavés avec du PBS et conservés dans de l'éthanol à 70%. La longueur des os a été mesurée par un pied à coulisse numérique (Festa).

A la fin de l'expérience, le sang a été collecté et laissé à coaguler à température ambiante pendant 2 heures. Les sérums ont été séparés par centrifugation (2 000 x g pendant 5 minutes, 4 °C) et conservés à -80 °C jusqu'à leur utilisation. Les niveaux d'IGF-1 ont été mesurés à l'aide du kit Mouse/Rat IGF-1 Quantikine ELISA (R&D systems) selon les instructions du fabricant.

Les données ont été analysées à l'aide du test statistique suivant: One way ANOVA avec Tukey's multiple comparisons test.

### 1.5 Résultats

Les résultats obtenus sont présentés sur les Figures 1 à 6 annexées.

La figure 1 représente l'évolution de la taille (en cm) en fonction du nombre de jours après la naissance pour chacune des compositions testées : composition A avec le MDP : courbe avec les carrés vides, composition B avec le murabutide : ronds vides et courbe avec les croix : composition placebo. La figure 2 représente l'évolution de la croissance relative (% de changement) en fonction du nombre de jours après la naissance pour chacune des compositions testées et en utilisant les mêmes symboles que pour la figure 1. La figure 3 représente l'évolution du taux de croissance (en cm/jour) pour chacune des compositions testées. La figure 4 représente la taille du fémur (en mm) pour chacune des compositions testées. La figure 5 représente la taille du tibia (en mm) pour chacune des compositions testées. La figure 6 représente le taux d'IGF-1 dans le sang (en ng/ml) pour chacune des compositions testées.

Ces résultats indiquent, une croissance relative, une taille totale et un taux de croissance significativement augmentés chez les souris ayant reçu la composition A comprenant du MDP ou la composition B comprenant du Murabutide par rapport aux souris ayant reçu la composition placebo. En particulier, le fémur des animaux traités par la composition A au MDP est significativement plus grand que celui des animaux traités avec la composition placebo avec une tendance positive du traitement murabutide qui n'atteint pas la significativité du test statistique. La longueur du tibia présente aussi une tendance positive de croissance accrue suite aux traitements MDP et murabutide par rapport aux traitements placebo mais qui n'atteint pas la significativité du test statistique. Enfin le taux circulant d'IGF1 est significativement augmenté chez les animaux ayant été traités au murabutide, et une tendance positive mais non significative est observée chez les animaux traités au MDP par rapport aux animaux traités au placebo.

### EXEMPLE 2 : Mise en évidence des effets de l'administration de mifamurtide dans un modèle de souris en sous-nutrition chronique

Dans cet exemple, on a testé les effets d'une composition conforme à la présente invention comprenant des quantités isomolaires d'un muramyl peptide le mifamurtide (Mw = 1237,50 g/mole) dans un modèle de souris en sous-nutrition chronique, à savoir : Mifamurtide 62,5 µg/jour dans 30 µL de placebo.

Cette composition a été comparée à une composition placebo renfermant uniquement de la maltodextrine (cryoprotectant) resuspendue dans 28,75 µL de tampon salin et 1,25 µL de DMSO.

### 2.1 Préparation des compositions

15 µg de cryoprotectant ont été dissous dans 1,25 µL de DMSO et 28,75 µL de tampon salin stérile pour générer une dose de composition placebo.

10 mg de Mifamurtide ont été resuspendus dans 200 µL de DMSO afin d'obtenir une solution à 50 µg/µL de Mifamurtide. 1,25 µL de cette solution ont été resuspendus dans 28,75 µL de composition placebo pour obtenir une composition ayant une concentration finale en Mifamurtide de 62,5 µg/ 30 µL (isomolaire de 25 µg/ 30 µL de MDP).

### 2.2 Préparation des animaux

Des souris de laboratoire C57BI/6j conventionnelles ont été élevées au Laboratoire de Gnotobiologie pendant plus de 10 générations. Les souris ont été maintenues dans des cages IVC (Tecniplast, Italie), exposées à des cycles lumière-obscurité de 12:12 heures, alimentées en eau du robinet et nourries *ad libitum* avec le régime alimentaire stérile V1124-300 (Ssniff Spezialdiäten GmbH) (irradié ~25 kGy, Bioster, République Tchèque). Les souris ont été accouplées et après l'accouchement, la taille de la portée a été réduite à 6 petits par mère. Au 21^{ème} jour, les souris mâles ont été sevrées avec le régime expérimental pauvre en protéines et en graisses (3,5 kcal/gramme, protéines : 4,1 % en poids, glucides : 78% en poids, Graisse : 2% en poids) et suivies régulièrement jusqu'à 56 jours après la naissance. Les souris sevrées avec le régime pauvre en protéines et en graisses ont été traitées 5 fois par semaine soit par la composition placebo, soit par la composition comprenant du mifamurtide telle que préparée ci-dessus au point 1.1 ci-dessus. A la fin de l'expérience, la nourriture et la litière ont été retirées à 8 heures du matin et les souris ont été sacrifiées après 5 heures de jeûne par inhalation d'isoflurane et dislocation cervicale. Tous les animaux ont été sacrifiés dans l'heure entre 13 et 14 heures.

### 2.3 Administration des compositions

Chacune des compositions a été administrée à la pipette sur la langue de la souris. La souris a été maintenue jusqu'à ce que la composition ait été avalée en totalité.

### 2.4 Description des tests effectués

Pour la mesure de la longueur du corps, les souris ont été brièvement anesthésiées à l'isoflurane (Piramal Healthcare, UK). La souris anesthésiée a été tenue par la queue et la longueur nez-anus a été mesurée à l'aide d'une règle. La longueur relative a été calculée comme un gain de longueur en %, où la longueur corporelle au jour 21 (sevrage) a été fixée à 100 %. Le taux de croissance de la longueur a été calculé en divisant le gain de longueur absolu du jour 21 au jour 56 par le nombre de jours (c'est-à-dire 35). Les os du fémur et du tibia ont été disséqués au moment du sacrifice, fixés dans du paraformaldéhyde à 4% - PBS pendant une nuit à 4°C, lavés avec du PBS et conservés dans de l'éthanol à 70%. La longueur des os a été mesurée par un pied à coulisse numérique (Festa).

A la fin de l'expérience, le sang a été collecté et laissé à coaguler à température ambiante pendant 2 heures. Les sérums ont été séparés par centrifugation (2 000 x g pendant 5 minutes, 4 °C) et conservés à -80 °C jusqu'à leur utilisation. Les niveaux d'IGF-1 ont été mesurés à l'aide du kit Mouse/Rat IGF-1 Quantikine ELISA (R&D systems) selon les instructions du fabricant.

Les données ont été analysées à l'aide du test statistique suivant: unpaired t-test.

### 2.5 Résultats

Les résultats sont présentés sur les Figures 7 à 12 annexées. La figure 7 représente l'évolution de la taille (en cm) en fonction du nombre de jours après la naissance pour chacune des compositions testées : composition avec le mifamurtide : courbe avec les triangles vides, composition placebo : courbe avec les croix. La figure 8 représente l'évolution de la croissance relative (% de changement) en fonction du nombre de jours après la naissance pour chacune des compositions testées et en utilisant les mêmes symboles que pour la figure 7. La figure 9 représente l'évolution du taux de croissance (en cm/jour) pour chacune des compositions testées. La figure 10 représente la taille du fémur (en mm) pour chacune des compositions testées. La figure 11 représente la taille du tibia (en mm) pour chacune des compositions testées. La figure 12 représente le taux d'IGF-1 dans le sang (en ng/ml) pour chacune des compositions testées.

Ces résultats indiquent, une croissance relative, une taille totale et un taux de croissance significativement augmentés chez les souris ayant reçu la composition contenant du Mifamurtide par rapport aux souris ayant reçu la composition placebo. La taille du fémur, du tibia et le taux circulant d'IGF1 des animaux traités avec la composition contenant du Mifamurtide sont significativement augmentés par rapport à ce qui est observé chez les animaux traités avec la composition placebo.

### EXEMPLE 3 : Mise en évidence des effets de l'administration d'une administration combinée de MDP et d'une bactérie à tropisme intestinal dans un modèle de souris en sous-nutrition chronique

Dans cet exemple, on a testé les effets de différentes compositions :
- une composition P (placebo) renfermant uniquement un cryoprotectant (15 µg de maltodextrine) resuspendu dans 30 µL de tampon salin stérile,
- une composition A conforme à la présente invention comprenant du MDP seul à raison de 25 µg/jour dans 30 µL de placebo,
- une composition AB conforme à la présente invention comprenant du MDP à raison de 25 µg/jour et des bactéries vivantes *Lactiplantibacillus plantarum* souche WJL à raison de 2.10⁸ CFU/jour dans 30 µL de placebo,
- une composition B comparative ne faisant pas partie de l'invention comprenant uniquement des bactéries *Lactiplantibacillus plantarum* souche WJL à raison de 2.10⁸ CFU/jour dans 30 µL de placebo.

### 3.1 Préparation des compositions

Les compositions P et A ont été préparées comme indiqué ci-dessus à l'exemple 1.

La composition AB a été préparée comme la composition A mais en ajoutant également 2.10⁸ CFU de la bactérie *Lactiplantibacillus plantarum* souche WJL dans les 30 µL de placebo en plus des 25 µg de MDP.

La composition B a été préparée en ajoutant uniquement 2.10⁸ CFU de la bactérie *Lactiplantibacillus plantarum* souche WJL dans les 30 µL de placebo.

### 3.2. Préparation des animaux

Les tests ont été réalisés sur des souris mâles avec chacune des compositions P, A, AB et B ci-dessus, selon le protocole indiqué à l'exemple 1, point 1.1 ci-dessus (période de 56 jours).

### 3.3. Administration des compositions

La dose quotidienne de traitement était de 30 µL de composition P (Placebo) ou de 25 µg de MDP seul dans 30 µL de placebo (composition A), ou de 25 µg MDP et de 2.10⁸ CFU de bactéries *Lactiplantibacillus plantarum* souche WJL dans 30 µL de placebo (composition AB) ou de 2.10⁸ CFU de bactéries *Lactiplantibacillus plantarum* souche WJL dans 30 µL de placebo (composition B).

Chacune des compositions a été administrée à la pipette sur la langue de la souris. La souris a été maintenue jusqu'à ce que la composition ait été avalée en totalité.

### 3.4. Description des tests effectués

La taille en longueur des souris a été mesurée de façon hebdomadaire pour chacun des lots de souris traitées par les compositions P, A, AB et B selon la méthode indiquée ci-dessus au point 1.4 de l'exemple 1.

Le gain sur la croissance en longueur à J56 (en %) des animaux traités par les compositions A, AB et B a été calculé par rapport la croissance en longueur mesurée à J56 sur les souris traitées par la composition placebo selon le protocole indiqué ci-dessus au point 1.4 de l'exemple 1.

Le taux d'IGF-1 circulant a été déterminé pour chacun des lots, selon le protocole indiqué ci-dessus à l'exemple 1, point 1.4. Le pourcentage d'amélioration du taux d'IGF-1 circulant pour chacune des compositions testées (A, AB et B) a ensuite été calculé par rapport au taux d'IGF-1 circulant mesuré sur les souris du lot traitées avec la composition placebo (100 %).

Les données numériques issues des mesures ont été analysées par des traitements statistiques adéquats (One way ANOVA avec Tukey's multiple comparisons test) afin de tester les effets de chacune des compositions A, AB et B et les effets cumulés éventuels entre traitement avec le MDP seul ou avec les bactéries *Lactiplantibacillus plantarum* souche WJL seules ou la combinaison MDP + bactéries *Lactiplantibacillus plantarum* souche WJL.

### 3.5. Résultats

Les résultats obtenus sont reportés sur les figures 13 et 14 annexées.

La figure 13 représente le % d'amélioration de croissance linéaire pour chacune des compositions testées comparativement au placebo.

La figure 14 représente le % d'amélioration taux d'IGF-1 circulant pour chacune des compositions testées comparativement au placebo.

Ces résultats confirment l'impact positif du MDP sur le taux de croissance linéaire relatif par rapport à la condition placebo. Ils montrent également que l'administration de la composition AB comprenant l'association de MDP et de bactéries *Lactiplantibacillus plantarum* souche WJL augmente significativement l'effet du traitement sur le taux de croissance linéaire relative par rapport à chacun des composants administrés seuls (Figure 13). Ce résultat est corroboré par l'étude du taux d'IGF-1 circulant chez les souris ayant reçu la composition AB, c'est-à-dire la combinaison MPD + bactéries *Lactiplantibacillus plantarum* souche WJL pour lesquels on observe un effet significativement plus marqué que lorsque chacun des composants est administré seul.

Par conséquent, la présence de bactéries à tropisme intestinal, telles que les bactéries *Lactiplantibacillus plantarum* souche WJL, permet d'améliorer de façon significative l'action des muramyl peptides sur la croissance squelettique.

## Revendications

1. Composition comprenant au moins un muramyl peptide, pour une utilisation dans le traitement des retards de croissance squelettique chez un humain ou un animal juvénile.

2. Composition pour une utilisation selon la revendication 1, **caractérisée en ce que** ledit au moins un muramyl peptide est choisi parmi le muramyl dipeptide et les analogues du muramyl dipeptide, lesdits analogues étant choisis parmi le mifamurtide, le murabutide, la 6-O-stearoyl-N-acetyl-muramyl-L-alanyl-D-isoglutamine, le muropeptide MurNAc-Ala-D-isoGln-L, et leurs mélanges.

3. Composition pour une utilisation selon la revendication 1 ou 2, **caractérisée en ce que** ledit au moins un muramyl peptide est le muramyl dipeptide.

4. Composition pour une utilisation selon l'une quelconque des revendications 1 à 3, **caractérisée en ce que** ledit au moins un muramyl peptide représente de 10 µg à 100 mg par dose unitaire de composition.

5. Composition pour une utilisation selon l'une quelconque des revendications 1 à 4, **caractérisée en ce que** ladite composition comprend en outre au moins un adjuvant permettant de favoriser la croissance, ledit adjuvant étant choisi parmi les bactéries appartenant aux familles *Lactobacillaceae, Streptoccaceae, Enterococcaceae, Leuconostocaceae,* et *Bifidobacteriaceae,* des constituants bactériens, des postbiotiques et leurs mélanges.

6. Composition pour une utilisation selon la revendication 5, **caractérisée en ce que** ledit adjuvant est choisi parmi les bactéries appartenant aux familles *Lactobacillaceae, Streptoccaceae, Enterococcaceae, Leuconostocaceae,* et *Bifidobacteriaceae.*

7. Composition pour une utilisation selon la revendication 5 ou 6, **caractérisée en ce que** lesdites souches sont choisies parmi les espèces *Lactobacillus plantarum, Lactobacillus fermentum,* et *Lactobacillus casei.*

8. Composition pour une utilisation selon l'une quelconque des revendications 5 à 7, **caractérisée en ce que** lesdites souches sont choisies parmi *L. plantarum WJL, L. plantarum* IGFL1, *L. plantarum* IGFL2, *L. plantarum* G821, *L*. *plantarum* NIZO2877, *L. casei* ATCC 393, *L. casei* L919, *L. paracasei* ATCC25302, *L. paracasei Shirota, L. fermentum* ATCC9338, *L. rhamnosus* L900, *L. rhamnosus* L908, *L. rhamnosus* GG, et leurs mélanges.

9. Composition pour une utilisation selon la revendication 5, **caractérisée en ce que** les constituants bactériens sont choisis parmi des bactéries inactivées, des lysats bactériens, des parois bactériennes purifiées et des fragments de parois bactériennes purifiées de bactéries ayant un tropisme intestinal, des peptidoglycanes et des acides téichoïques.

10. Composition pour une utilisation selon l'une quelconque des revendications précédentes, **caractérisée en ce qu'**elle renferme, à titre d'ingrédient additionnel, un ou plusieurs compléments alimentaires et/ou nutriments choisis parmi les glucides, les lipides, des peptides autres que les muramyl peptides, les protéines, les acides gras, les acides aminés, les vitamines, les minéraux, et les prébiotiques.

11. Composition pour une utilisation selon l'une quelconque des revendications précédentes, **caractérisée en ce que** le sujet humain ou animal est en sous-nutrition chronique due à une carence en protéines.

12. Utilisation non thérapeutique d'une composition comprenant au moins un muramyl peptide et au moins une bactérie appartenant aux familles *Lactobacillaceae, Streptoccaceae, Enterococcaceae, Leuconostocaceae,* et *Bifidobacteriaceae* à titre d'adjuvant, ladite bactérie étant vivante ou inactivée, pour favoriser la croissance squelettique chez un sujet animal ou humain.

## Patentansprüche

1. Zusammensetzung, die wenigstens ein Muramylpeptid umfasst, zur Verwendung bei der Behandlung von Verzögerungen des Skelettwachstums bei einem jungen Menschen oder einem jungen Tier.

2. Zusammensetzung zur Verwendung gemäß Anspruch 1, **dadurch gekennzeichnet, dass** das wenigstens eine Muramylpeptid aus dem Muramyldipeptid und den Analoga des Muramyldipeptids ausgewählt ist, wobei die Analoga aus Mifamurtid, Murabutid, 6-O-Stearoyl-N-acetylmuramyl-L-alanyl-D-isoglutamin, dem Muropeptid MurNAc-Ala-D-isoGln-L und deren Gemischen ausgewählt sind.

3. Zusammensetzung zur Verwendung gemäß Anspruch 1 oder 2, **dadurch gekennzeichnet, dass** das wenigstens eine Muramylpeptid das Muramyldipeptid ist.

4. Zusammensetzung zur Verwendung gemäß einem der Ansprüche 1 bis 3, **dadurch gekennzeichnet, dass** das wenigstens eine Muramylpeptid 10 µg bis 100 mg pro Dosiseinheit der Zusammensetzung ausmacht.

5. Zusammensetzung zur Verwendung gemäß einem der Ansprüche 1 bis 4, **dadurch gekennzeichnet, dass** die Zusammensetzung weiterhin wenigstens ein Adjuvans umfasst, das es ermöglicht, das Wachstum zu fördern, wobei das Adjuvans aus Bakterien, die zu den Familien Lactobacillaceae, Streptococcaceae, Enterococcaceae, Leuconostocaceae und Bifidobacteriaceae gehören, Bestandteilen der Bakterien, Postbiotika und deren Gemischen ausgewählt ist.

6. Zusammensetzung zur Verwendung gemäß Anspruch 5, **dadurch gekennzeichnet, dass** das Adjuvans aus Bakterien, die zu den Familien Lactobacillaceae, Streptococcaceae, Enterococcaceae, Leuconostocaceae und Bifidobacteriaceae gehören, ausgewählt ist.

7. Zusammensetzung zur Verwendung gemäß Anspruch 5 oder 6, **dadurch gekennzeichnet, dass** die Stämme aus den Spezies *Lactobacillus plantarum, Lactobacillus fermentum* und *Lactobacillus casei* ausgewählt sind.

8. Zusammensetzung zur Verwendung gemäß einem der Ansprüche 5 bis 7, **dadurch gekennzeichnet, dass** die die Stämme aus *L. plantarum* WJL, *L. plantarum* IGFL1, *L. plantarum* IGFL2, *L. plantarum* G821, *L. plantarum* NIZO2877, *L. casei* ATCC 393, *L. casei* L919, *L. paracasei* ATCC 25302, *L*. *paracasei* Shirota, *L. fermentum* ATCC 9338, *L. rhamnosus* L900, *L*. *rhamnosus* L908, *L. rhamnosus* GG und deren Gemischen ausgewählt sind.

9. Zusammensetzung zur Verwendung gemäß Anspruch 5, **dadurch gekennzeichnet, dass** die Bestandteile der Bakterien aus inaktivierten Bakterien, Bakterienlysaten, gereinigten bakteriellen Zellwänden und Fragmenten von gereinigten bakteriellen Zellwänden von Bakterien, die einen Darmtropismus aufweisen, Peptidoglykanen und Teichonsäuren ausgewählt sind.

10. Zusammensetzung zur Verwendung gemäß einem der vorstehenden Ansprüche, **dadurch gekennzeichnet, dass** sie als zusätzlichen Bestandteil ein oder mehrere Nahrungsergänzungsmittel und/oder Nährstoffe, die aus Kohlenhydraten, Lipiden, anderen Peptiden als den Muramylpeptiden, Proteinen, Fettsäuren, Aminosäuren, Vitaminen, Mineralstoffen und Präbiotika ausgewählt sind, umfasst.

11. Zusammensetzung zur Verwendung gemäß einem der vorstehenden Ansprüche, **dadurch gekennzeichnet, dass** der menschliche oder tierische Patient wegen Proteinmangels chronisch unterernährt ist.

12. Nichttherapeutische Verwendung einer Zusammensetzung, die wenigstens ein Muramylpeptid und wenigstens ein Bakterium, das zu den Familien Lactobacillaceae, Streptococcaceae, Enterococcaceae, Leuconostocaceae und Bifidobacteriaceae gehört, als Adjuvans umfasst, wobei das Bakterium lebend oder inaktiviert ist, um das Skelettwachstum bei einem Tier oder Menschen zu fördern.

## Claims

1. A composition comprising at least one muramyl peptide, for use in the treatment of skeletal growth retardation in a juvenile human or animal.

2. The composition for use according to claim 1, **characterised in that** said at least one muramyl peptide is selected from muramyl dipeptide and muramyl dipeptide analogues, said analogues being selected from mifamurtide, murabutide, 6-O-stearoyl-N-acetyl-muramyl-L-alanyl-D-isoglutamine, muropeptide MurNAc-Ala-D-isoGln-L, and mixtures thereof.

3. The composition for use according to claim 1 or 2, **characterised in that** said at least one muramyl peptide is muramyl dipeptide.

4. The composition for use according to any of claims 1 to 3, **characterised in that** said at least one muramyl peptide represents from 10 µg to 100 mg per unit dose of composition.

5. The composition for use according to any of claims 1 to 4, **characterised in that** said composition additionally comprises at least one adjuvant for promoting growth, said adjuvant being selected from bacteria belonging to the *Lactobacillaceae, Streptoccaceae, Enterococcaceae, Leuconostocaceae* and *Bifidobacteriaceae* families, bacterial constituents, postbiotics and mixtures thereof.

6. The composition for use according to claim 5, **characterised in that** said adjuvant is selected from bacteria belonging to the *Lactobacillaceae, Streptoccaceae, Enterococcaceae, Leuconostocaceae* and *Bifidobacteriaceae* families.

7. The composition for use according to claim 5 or 6, **characterised in that** said strains are selected from the *Lactobacillus plantarum, Lactobacillus fermentum* and *Lactobacillus casei* species.

8. The composition for use according to any of claims 5 to 7, **characterised in that** said strains are selected from *L. plantarum* WJL, *L. plantarum* IGFL1, *L. plantarum* IGFL2, *L. plantarum* G821, *L. plantarum* NIZ02877, L. *casei* ATCC 393, L. *casei* L919, L. *paracasei* ATCC25302, L. *paracasei Shirota, L. fermentum* ATCC9338, L. *rhamnosus* L900, *L*. *rhamnosus* L908, L. *rhamnosus* GG, and mixtures thereof.

9. The composition for use according to claim 5, **characterised in that** the bacterial constituents are selected from inactivated bacteria, bacterial lysates, purified bacterial walls and purified bacterial wall fragments of bacteria with intestinal tropism, peptidoglycans and teichoic acids.

10. The composition for use according to any of the preceding claims, **characterised in that** it contains, as an additional ingredient, one or more food supplements and/or nutrients selected from carbohydrates, lipids, peptides other than muramyl peptides, proteins, fatty acids, amino acids, vitamins, minerals and prebiotics.

11. The composition for use according to any of the preceding claims, **characterised in that** the subject is a chronically undernourished human or animal subject due to protein deficiency.

12. Non therapeutic use of a composition comprising at least one muramyl peptide and at least one bacteria belonging to the *Lactobacillaceae, Streptoccaceae, Enterococcaceae, Leuconostocaceae* and *Bifidobacteriaceae* families as an adjuvant, said bacteria being alive or inactivated, for promoting skeletal growth in an animal or human subject.
